# EUROPEAN PATENT SPECIFICATION

(11) **EP 1 755 540 B1**
(45) Date of publication and mention of the grant of the patent: **06.10.2010**
(21) Application number: 05762416.5
(22) Date of filing: 13.06.2005
(51) Int. Cl.: A61Q 11/00, A61K 8/73, A61K 8/25, A61K 8/26

(54) **ORAL CARE COMPOSITIONS**
MUNDPFLEGEZUSAMMENSETZUNGEN
COMPOSITIONS DE SOINS BUCCAUX

(30) Priority: 18.06.2004 US 871659
(43) Date of publication of application: 28.02.2007
(73) Proprietor: The Gillette Company, Boston, Massachusetts 02199 (US)
(72) Inventor: DODD, Kenneth, T., Upton, MA 01568 (US); GURGE, Ronald, M., Franklin, MA 02038 (US); TYNDALL, David, V., Medway, MA 02053 (US)
(74) Representative: Clemo, Nicholas Graham
(86) International application number: PCT/US2005/020882
(87) International publication number: WO 2006/009679

(56) References cited:
- EP-A- 0 565 401
- WO-A-99/63961
- WO-A-99/63961
- WO-A-2004/032674
- FR-A- 2 556 961
- FR-A- 2 556 962
- US-A- 5 324 505
- US-A- 5 833 956
- US-A- 5 833 956
- DATABASE CAPLUS [Online] CHEMICAL ABSTRACTS SERVICE, COLUMBUS, OHIO, US; XP002501241 retrieved from STN Database accession no. 1996:399425

## Description

This invention relates to an oral care composition which can be dispensed from a dispensing toothbrush.

The current oral care market presents the consumer with a variety of dentifrice, e.g., toothpaste, options for use on either conventional manual or power toothbrushes. The offerings range from tooth powders to clear gels to the more common opaque pastes. Dentifrice is widely recognized as important in oral health, especially for the reduction of caries. Most compositions contain active ingredients for cleaning the oral cavity, such as abrasives and surfactants, anticavity ingredients, such as fluoride, tartar control ingredients, pH buffers, whitening agents, humectants, and a variety of actives for treating an array of oral health problems. Dentifrice formulations vary tremendously depending upon a variety of factors, including ingredient compatibility, cost, and the desired benefits and quality of the product.

In most instances, a dentifrice is a three-phase system, including a continuous phase, a dispersed phase, and a solid phase. While the appropriate blending and relative composition of these components is important to obtain a stable system, the flow characteristics or Rheology of the formulation is important for consumer acceptance. For example, it is generally desirable that a dentifrice does not separate with aging, is easy to dispense from a toothpaste tube, demonstrates good ribbon properties without strings, holds form on the top of a toothbrush, and distributes evenly and smoothly over the teeth.

Oral care compositions such as dentifrice are described below. Generally, the oral care compositions described herein have rheological properties that provide desirable dispensing characteristics when dispensed from a dispensing toothbrush. The inventors have found that, when oral care compositions are dispensed using a dispensing toothbrush, some of the traditional rheological characteristics associated with dentifrice are not necessary. For example, when the oral care composition is delivered while the head of the dispensing toothbrush is in the user's mouth, it is not generally necessary that the oral care composition hold its shape on the brush. On the other hand, it is generally desirable that the oral care composition flow easily through a mechanical pumping system, thus providing a continuous and even dispensing of the oral care composition from the dispensing toothbrush.

The present invention features oral care compositions having rheological properties that render the compositions suitable for use in dispensing toothbrushes. Some preferred compositions also exhibit good sensory characteristics, e.g., flavor intensity and impact, tingle, ease of rinsing and ease of spreading, and good efficacy, e.g., cleaning, tartar removal, whitening and anti-caries protection. The compositions also generally remain stable during aging, e.g., exhibiting little or no phase separation after 3 months at accelerated aging conditions of 40°C and 45% Relative humidity.

The invention features an oral care composition that includes a carrier and a binder.

The binder includes hydrous sodium lithium magnesium silicate and sodium carboxymethylcellulose.

In some instances, the binder includes a synthetic clay, for example a laponite, a hectorite, or a bentonite.

The oral care composition also includes sodium lauryl sulphate, cocamidopropyl betaine, and D-glucopyranoside C₁₀₋₁₆ alkyl oligomeric.

In some instances, the oral care composition includes a thickening agent. An example of a thickening agent includes thickening silica. In some instances, the oral care composition includes between 4% to 8%, for example 6% thickening silica. In other instances, the thickening agent includes a polymer, for example an alginate, cellulose, a cellulose derivative, or carageenan. In some instances the thickening agent is carageenan. For example, the oral care composition can include between 0.1% and 2.0% by weight of carageenan.

In some instances, the oral care composition includes a polishing agent. Examples of polishing agents include sodium bicarbonate, water-colloidal silica, percipiated silicas, sodium aluminosilicate, silica grades containing alumina, hydrated alumina, dicalcium phosphate, insoluable sodium metaphosphate, calcium carbonate, magnesium, trimagnesium phophate or combinations thereof. In some instances the oral care composition includes abrasive silica or hydrated silica.

In some instances the oral care composition includes xanthan gum and hydrous sodium lithium magnesium silicate. For example, the oral care composition can include between 0.1% to 2.0% by weight combined of xanthan gum and hydrous sodium lithium magnesium silicate. In some instances, the oral care composition can have a ratio of the xanthan gum to hydrous sodium lithium magnesium silicate of between 0.5:1 to 2:1.

In some instances, the active ingredient includes an antimicrobial agent, a phenolic compound, an anti-inflammatory agent, an anti-caries agent, a plaque buffer, a desensitizing agent, an anti-calculus agent, a biomolecule, or combinations thereof.

In some instances, the carrier includes water, polyethylene glycol, polypropylene glycol, glycerin or combinations thereof.

In some instances, the oral care composition has a Newtonian viscosity at 50 to 400 Pa of less than 1.0 Pa.s, for example a Newtonian viscosity at 50 to 400 Pa of less than 0.5 Pa.s.

In some instances, the oral care composition includes a humectant. Examples of a suitable humectant include glycerin, sorbitol or a combination thereof.

In some instances, the oral care composition includes a salt. Examples of salts include sodium saccharin, tetrasodium pyrophosphate, sodium hydroxide and combinations thereof.

The oral care composition can be, for example, a dentifrice.

In some instances, the oral care composition includes 0.1 to 1.0% hydrous sodium lithium magnesium silicate, 0.1 to 1.0% sodium carboxymethylcellulose, 1.0 to 2.0% sodium lauryl sulphate, 0.2 to 1.2% cocamidopropyl betaine, and 0.1 to 1.0% D-glucopyranoside C₁₀₋₁₆ alkyl oligomeric. For example, the oral care composition can include 0.4 to 0.6 % hydrous sodium lithium magnesium silicate, 0.35 to 0.40% sodium carboxymethylcellulose, 1.5 to 1.6% sodium lauryl sulphate, 0.6 to 0.8% cocamidopropyl betaine, and 0.4 to 0.6% D-glucopyranoside C₁₀₋₁₆ alkyl oligomeric.

In some instances, the oral care composition includes an abrasive.

The term "Newtonian viscosity" refers to the viscosity of a Newtonian fluid. A Newtonian fluid is one in which the viscosity does not vary with the deformation rate or time. In other words the fluid flow and the rate of shear are generally proportional to the applied stress. Many liquids demonstrate this behavior under a wide range of shear rates while others show this type of behavior under only a limited range of shear rates.

Dentifrice formulations exhibit Bingham plastic behavior where the formulation shows little or no deformation up to a certain level of stress. This yield stress or "yield point" refers to the stress limit at which permanent deformation takes place. The deformation can take place, for example, by undergoing flow.

As viscosity is defined in terms of the shear stress versus the strain rate, the "shear slope" is the rate of change of the viscosity after the fluid begins to flow.

The term "dentifrice" refers to a substance, such as a tooth powder, clear gel or opaque paste, for cleaning teeth.

The term "active ingredient" refers to any material included in an oral care composition that provides a benefit to the user. Examples of such benefits include anti-caries activity, anti-halitosis activity, anti-microbial activity, tooth whitening activity, etc.

Unless otherwise noted, all percentages given are percent by weight.

Oral care compositions such as dentifrice can be dispensed in a variety of ways. For example, dentifrice can be dispensed from a tube, or alternatively, directly from a toothbrush. As discussed above, the inventors have found that, in many instances, the rheological properties that are desirable for a dentifrice dispensed from a tube are different from the properties desirable in a dentifrice dispensed directly from a toothbrush.

Applicants have also discovered combinations of materials that provide oral care compositions having desirable rheological properties, especially when used in combination with a dispensing toothbrush.

### Binder

The binder system, generally, is a primary factor that determines the rheological characteristics of the oral care composition. The binder also acts to keep any solid phase of the oral care composition suspended in the formulation, thus preventing separation of the solid phase portion of the oral care composition from the liquid phase portion of the formulation. Additionally, the binder can provide body or thickness to the oral care composition. Thus, in some instances, a binder can also provide a thickening function to an oral care composition.

The binder is chosen to provide the oral care composition with shear-thinning properties, i.e., a rapid loss in viscosity as a shear force is applied to the oral care composition, that allow the oral care composition to flow easily through a dispensing toothbrush (e.g., through a mechanical pumping assembly such as a peristaltic pumps). A combination of binders are used together to provide a binder system with desirable shear-thinning properties.

Examples of binders that can provide desirable rheological characteristics include sodium carboxymethyl-cellulose, cellulose ether, xanthan gum, carrageenan, sodium alginate, carbopol, hydrous sodium lithium magnesium silicate. In each instance, these binders can also be used in combination binder systems to provide an oral care composition with desirable rheological characteristics.

The oral care composition of the present invention has a binder system that includes a combination of hydrous sodium lithium magnesium silicate (commonly referred to as "laponite") and sodium carboxymethylcellulose (CMC). In some instances, the oral care composition includes at least 0.15% hydrous sodium lithium magnesium silicate by weight, based on the total weight of the composition (e.g., at least 0.20%, 0.25%, 0.30%, 0.35%, or 0.40%) and at most 1% hydrous sodium lithium magnesium silicate (e.g., at most 0.95%, 0.90%, 0.85%, 0.80%, 0.75%, 0.70%, or 0.65%). The oral care composition also includes at least 0.08% sodium carboxymethylcellulose (e.g., at least 0.10%, 0.12%, 0.13%, 0.15%, 0.20% or 0.25%) and at most 0.65% sodium carboxymethylcellulose (e.g., at most 0.60%, 0.55%, 0.50%, 0.45%, 0.40%, or 0.35%).

In some instances, thickening silica (e.g., fumed silica) is included in the oral care compositions in addition to the hydrous sodium lithium magnesium silicate and sodium carboxymethylcellulose binding system. For example, the oral care composition can include at least 2% (e.g., at least 3%, at least 4%, at least 5%, or at least 6%) and at most 10% (e.g., at most 9%, at most 8%, at most 7%, or at most 6%) of a thickening silica.

Preferably, the ratio of hydrous lithium magnesium silicate to sodium carboxymethylcellulose is in the range of 1:1 to 3:2. If thickening silica is included, the ratio of hydrous lithium magnesium silicate to sodium carboxymethylcellulose to thickening silica can be in the range of 1:1:16 to 3:3:32.

Table 1 below provides amounts of hydrous sodium lithium magnesium silicate, sodium carboxymethylcellulose, and thickening silica used in certain embodiments of an oral care composition.

**Table 1**

| **Oral care composition formulations** | |
|---|---|
| Ingredients | Concentration in percent by weight |
| Hydrous Sodium Lithium Magnesium Silicate | 0.25 - 0.75 |
| Sodium Carboxymethylcellulose | 0.1 - 0.50 |
| Thickening Silica | 4 - 8 |

Oral care compositions having various percent by weight concentrations of hydrous sodium lithium magnesium silicate, sodium carboxymethylcellulose and thickening silica (e.g., SILOX^{™} fumed silica) were tested to determine rheological properties including the yield point Pa and shear slope Pa/sec⁻¹ as described above. Examples of oral care compositions having various combinations of hydrous sodium lithium magnesium silicate (i.e., laponite), sodium carboxymethylcellulose (i.e., CMC) and SILOX^{™} silica are shown in Table 2 below together with the yield point and shear slope of each formulation. A preferred yield point would occur between 10 and 80 Pa coupled with a shear slope ranging form 0.06 - 0.11. For those skilled in the art, the selection of an appropiate range is generally based on the amount of solid phase, the robustness of the pump system and the length and size of the conveyance tubing.

**Table 2**

| **Rheological Properties of Oral Care Compositions** **Including CMC, Laponite, and Silica** | | | | |
|---|---|---|---|---|
| **CMC (% by wt.)** | **Laponite (% by wt.)** | **Silica (% by wt.)** | **Yield point (Pa)** | **Shear Slope (Pa/sec⁻¹)** |
| 0.13 | 0.50 | 4.00 | 1.00 | 0.08 |
| 0.13 | 0.75 | 4.00 | 10.00 | 0.10 |
| 0.25 | 0.25 | 4.00 | 6.31 | 0.13 |
| 0.25 | 0.50 | 4.00 | 25.12 | 0.07 |
| 0.25 | 0.75 | 4.00 | 39.81 | 0.08 |
| 0.38 | 0.25 | 4.00 | 25.12 | 0.09 |
| 0.38 | 0.50 | 4.00 | 31.62 | 0.08 |
| 0.38 | 0.75 | 4.00 | 79.24 | 0.06 |
| 0.50 | 0.25 | 4.00 | 50.12 | 0.07 |
| 0.50 | 0.75 | 4.00 | 79.43 | 0.07 |
| 0.13 | 0.50 | 8.00 | 10.00 | 0.10 |
| 0.13 | 0.75 | 8.00 | 19.91 | 0.11 |
| 0.25 | 0.25 | 8.00 | 25.12 | 0.07 |
| 0.25 | 0.50 | 8.00 | 25.12 | 0.11 |
| 0.25 | 0.75 | 8.00 | 50.12 | 0.06 |
| 0.38 | 0.25 | 8.00 | 50.12 | 0.06 |
| 0.38 | 0.50 | 8.00 | 50.00 | 0.08 |
| 0.38 | 0.75 | 8.00 | 79.24 | 0.06 |
| 0.50 | 0.25 | 8.00 | 63.09 | 0.07 |
| 0.50 | 0.75 | 8.00 | 79.43 | 0.06 |

### Surfactants/Detergents

The oral care composition (e.g., a dentifrice) includes surfactants or detergents to provide a desirable foaming quality to the oral care composition.

Applicants have discovered that the combination of sodium lauryl sulfate, cocamidopropyl betaine, and D-glucopyranoside C10-C16 alkyl oligomeric provide, give desirable foam characteristics to the oral care composition. In some instances, the oral care composition includes at least 0.8% sodium lauryl sulphate (e.g., at least 1.0%, 1.2%, 1.4%, or 1.5%) and at most 2.2% sodium lauryl sulphate (e.g., at most 2.0%, 1.8%, or 1.6%). In some instances, the oral care composition includes at least 0.4% cocamidopropyl betaine (e.g., at least 0.5%, 0.6%, 0.7%, or 0.8%) and at most 1.2% cocamidopropyl betaine (e.g., at most 1.0%, or 0.8%). In some instances, the oral care composition includes 0.4% D-glucopyranoside C10-C16 alkyl oligomeric (e.g., at least 0.5%, 0.6%, 0.7%, or 0.8%) and at most 1.2% D-glucopyranoside C10-C16 alkyl oligomeric (e.g., at most 1.0% or 0.8%). For example, in some instances, the oral care composition includes 1.57% sodium lauryl sulphate, 0.70% cocamidopropyl betaine, and 0.51% D-glucopyranoside C10-C16 alkyl oligomeric. In general, an oral care composition can include, for a three surfactant blend of sodium lauryl sulphate, cocamidopropyl betaine, D-glucopyranoside C10-C16 alkyl oligomeric, a ratio of surfactant components of 3:0.5:0. respectively.

### Thickening agents

In some instances, the oral care compositions include a thickening agent. Examples of thickening agents include thickening silica (discussed above), polymers, clays, and combinations thereof. Applicants have discovered that thickening silica, for example, SILODENT 15 hydrated silica, in the amount between 4% to 8% by weight (e.g., 6%) provide desirable in- mouth characteristics. The phrase "in-mouth characteritics" as described herein relates to the body and thickness of the composition as it foams in the mouth of a user.

### Polishing agents

In many instances, the oral care compositions include a polishing agent. In some instances, the polishing agent is an abrasive. Examples of polishing agents include carbonates (e.g., sodium bicarbonate, calcium carbonate) water-colloidal silica, precipitated silicas (e.g., hydrated silica), sodium aluminosilicates, silica grades containing alumina, hydrated alumina, dicalcium phosphates, insoluble sodium metaphosphate, and magnesiums (e.g., trimagnesium phosphate). A suitable amount of polishing agent is an amount that safely provides good polishing and cleaning and which, when combined with other ingredients in the oral care composition gives a smooth, flowable, and not excessively gritty composition. In general, when polishing agents are included in the oral care composition, they are present in an amount from 5% to 50% by weight (e.g., from 5% to 35%, or from 7% to 25%).

### Carriers

The oral care compositions include a carrier. Examples of carriers include water, polyethylene glycol, glycerin, polypropylene glycol, starches, sucrose, alcohols (e.g., methanol, ethanol, isopropanol, etc.), or combinations thereof. Examples of combinations include various water and alcohol combinations and various polyethylene glycol and polypropylene glycol combinations. In general, the amount of carrier in an oral care composition is determined based on the concentration of the binder system along with the amount of dissolved salts, surfactants, and dispersed phase.

### Humectants

In many instances, the oral care compositions include a humectant. Generally, humectants are polyols. Examples of humectants include glycerin, sorbitol propyleneglycol, xylitol, lactitol, polypropylene glycol, polyethylene glycol, hydrogenated corn syrup and mixtures thereof. In general, the humectants can include from 10% to 60% by weight of the oral care composition.

### Buffers and/or Salts

In many instances the oral care composition includes one or more buffers or salts. Examples of buffers and salts include primary, secondary, or tertiary alkali metal phosphates, citric acid, sodium citrate, sodium saccharin, tetrasodium pyrophosphate, sodium hydroxide, and the like.

### Active ingredients

Oral care compositions can be put to a variety of uses, for example, to prevent cavities, to whiten teeth, to freshen breath, to deliver oral medication, and to provide other therapeutic and cosmetic benefits. Accordingly, the oral care compositions can include one or more of a variety of active ingredients. Examples of active ingredients include the following: anti-caries agents (e.g., water soluble fluoride salts, fluorosilicates, fluorozirconates, fluorostannites, fluoroborates, fluorotitanates, fluorogermanates, mixed halides and casine); anti-tarter agents; anti-calculus agents (e.g. alkali-metal pyrophosphates, hypophosphite-containing polymers, organic phosphocitrates, phosphocitrates, polyphosphates); anti-bacterial agents (e.g., bacteriocins, antibodies, enzymes); anti-bacterial enhancing agents; anti-microbial agents (e.g., Triclosan, chlorhexidine, copper-, zinc- and stannous salts such as zinc citrate, zinc sulfate, zinc glycinate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenbis-(4-chloro-6-bromophenol)); desensitizing agents (e.g., potassium citrate, potassium chloride, potassium tartrate, potassium bicarbonate, potassium oxalate, potassium nitrate and strontium salts); whitening agents (e.g., bleaching agents such as peroxy compounds, e.g. potassium peroxydiphosphate); anti-plaque agents; gum protecting agents (e.g., vegetable oils such as sunflower oil, rape seed oil, soybean oil and safflower oil, and other oils such as silicone oils and hydrocarbon oils). The gum protection agent may be an agent capable of improving the permeability barrier of the gums. Other active ingredients include wound healing agents (e.g., urea, allantoin, panthenol, alkali metal thiocyanates, chamomile-based actives and acetylsalicylic acid derivatives, ibuprofen, flurbiprofen, aspirin, indomethacin etc.); tooth buffering agents; reminieralization agents; anti-inflammatory agents; anti-malodor agent; breath freashing agents; and agents for the treatment of oral conditions such as gingivitis or periodontitis.

### Other ingredients

In some instances, the oral care compositions include effervescing systems such as sodium bicarbonate citric acid systems, or color change systems.

The oral care compositions may also include one or more of the following: phenolic compounds (e.g., phenol and its homologues, including 2-methyl-phenol, 3-methyl-phenol. 4-methyl-phenol, 4-ethyl-phenol, 2,4-dimethol-phenol, and 3,4-dimethol -phenol); sweetening agents (e.g., sodium saccharin, sodium cyclamate, sucrose, lactose, maltose, and fructose); flavors (e.g., peppermint oil, spearmint oil, eucalyptus oil, aniseed oil, fennel oil, caraway oil, methyl acetate, cinnamaldehyde, anethol, vanillin, thymol and other natural or nature-identical essential oils or synthetic flavors); preservatives (e.g., p-hydroxybenzoic acid methyl, ethyl, or propyl ester, sodium sorbate, sodium benzoate, bromochlorophene, triclosan, hexetidine, phenyl silicylate, biguanides, and peroxides); opacifying and coloring agents such as titanium dioxide or F D & C dyes; and vitamins such as retinol, tocopherol or ascorbic acid.

### Rheological Properties

The shear-thinning properties and other rheological properties of an oral care composition may be evaluated, for example, by measuring the yield point and shear slope of the composition, and/or by measuring the Newtonian viscosity of the composition. In one instance, the rheological properties of the oral care composition for shear slopes from 0 - 0.4 Pa/sec are defined by a yield point of y Pa and a shear slope of x Pa/sec, with the ratio of yield point y to shear slope x for formulations being less than y/(74 -370x + 470x²). The rheological properties of the oral care composition may also be defined by a Newtonian viscosity, for example, between 50 and 400 Pa of less than 1.0 Pa.s. In some instances, where the yeild point is less than 5 Pa, the shear slope is greater than 0.2 Pa/sec.

The rheological characteristics (e.g., flow characteristics) can be measured, for example, using a Rhometrics controlled stress rheometer. For example, measurements can be made at a constant temperature of 20 °C using a cone and plate geometry. The cone is 45 mm in diameter with a gap setting of 0.051 mm. Yield stress and shear slope are measured, and a flow curve measuring formulation viscosity is determined as a function of the applied stress. From the curve information concerning the thickness of the product and the strength of the structure at rest (zero shear viscosity and yield stress) the ease at which the structure will break down (i.e., shear thinning) can be determined. Additionally, creep measurements can be made to determine the Newtonian viscosity of the compositions at 50 Pa and 400 Pa.

### Dispensing Toothbrushes

The oral care compositions may be used in dispensing toothbrushes, for example those described in copending U.S. Applications: "ORAL CARE DEVICE", Attorney Docket No. 00216-629001, filed June 3, 2004; "ORAL CARE DEVICE", Attorney Docket No. 00216-640001, and filed June 3, 2004; "ORAL CARE DEVICE", Attorney Docket No. 00216-641001, filed June 3, 2004. Such toothbrushes include mechanical pumps, e.g., pumps that operate by peristaltic action.

### EXAMPLES

### Example 1

The dentifrice formulation shown in Table 3 is an example of a dentifrice having desirable rheological properties for dispensing through a dispensing toothbrush. The dispensing dentifrice has a 0 shear viscosity of 2.05E+04 Pa×s, a yield point of 18.85 Pa, and a shear slope of 0.104 Pa/sec. Additionally, the dispensing dentifrice has a Newtonian viscosity at 50 Pa of 1.45E+01 Pa×s and a Newtonian viscosity at 400 Pa of 0.00E+00 Paxs. All measurements were taken using the Rheometrics controlled stress rheometer as described above.

**Table 3**

| **Formulation of Dispensing Dentifrice** | |
|---|---|
| **Raw Material** | **Concentration (% by wt.)** |
| Hydrous Sodium Lithium Magnesium Silicate | 0.50 |
| Sodium Carboxymethylcellulose | 0.38 |
| Whitening Silica | 10 |
| Thickening Silica | 6 |
| Glycerine | 10 |
| Sorbitol | 30 |
| Sodium Saccharin | 0.30 |
| Tetrasodiumpyrophosphate | 0.025 |
| Sodium Hydroxide | 0.0625 |
| Sodium Lauryl Sulphate | 1.57 |
| Cocamidopropyl Betaine | 0.70 |
| D-Glucopyranoside, C10-16 Alkyl, Oligomeric | 0.51 |
| Flavor | 1.00 |
| Purified Water | 38.9525 |

Additional examples of dentifrice formulations are provided in Tables 4-7 below.

The oral care compositions described in Tables 4-7 are made as described in a two phase system as described.

Phase A: Approximately 3/4 of the water is added to a kettle, with the remaining portion retained for Phase B. Sorbitol is added to the water and mixed thoroughly, and the mixture is meated to 80 °C. Once the water has reached 80 °C the laponite D is slowly added and mixed for 15 minutes. The CMC is then added to glycerine and mixed until a uniform dispersion is formed. The CMC and glycerine blend is then added to the kettle and mixed for 30 minutes. Salts are then added (i.e., Saccharin, tetrasodiumpyrophosphate, and sodium hydroxide) to the kettle and mixed until blended. Once the above three ingredients are blended, the kettle is removed and cooled, at which point the whitening silica is added. Once the addition is complete the composition is mixed for min 30 minutes or until uniformly blended.

**Phase B:** Using remaining water from above, the surfactants (SLS, Cocamidopropyl Betaine, and D-Glucopyranoside, C10-16 Alkyl, Oligomeric) are added and mixed for 30 minutes or until blended. The thickening silica is then added and mixed for 15 minutes or until uniformly dispersed. After the thickening silica is uniformly dispersed, the flavor is added and mixed for 10 minutes or until uniformly blended. Once the kettle temperature is below 40 °C, phase B is added to the kettle containing phase A and mixed for minimum of 30 minutes or until uniformly blended.

**Table 4**

| **Dentifrice formulations** | | |
|---|---|---|
| **Raw material** | | |
| DI Water | 38.95% | 38.70% |
| Hydrous Sodium Lithium Magnesium Silicate | 0.50% | 0.50% |
| Sodium carboxymethylcellulose | 0.38% | 0.38% |
| Xanthan gum | | |
| Carrageenan Benvisco LB-2105 | | |
| Abrasive Silica | 10% | 10% |
| Thickening Silica | 6% | 6% |
| Sodium Fluoride | | 0.25% |
| Glycerine | 10% | 10% |
| Sorbitol | 30% | 30% |
| Sodium Saccharin | 0.30% | 0.30% |
| Tetrasodiumpyrophosphate | 0.03% | 0.03% |
| Sodium Hydroxide | 0.06% | 0.06% |
| Sodium Lauryl Sulphate | 1.57% | 1.57% |
| Cocamidopropyl Betaine | 0.70% | 0.70% |
| D-Glucopyranoside, C10-16 Alkyl, Oligomeric | 0.51% | 0.51% |
| Phorphoric Acid | | |
| Monobasic Sodium Phosphate (USP)(Anhydrous) | | |
| Disodium Edetate USP | | |
| Hydrogen Peroxide 35% (as supplied) | | |
| Calcium Peroxide | | |
| Nipagin M | | |
| Niipasol M | | |
| Glycosperse L-20 | | |
| Flavor | 1.00% | 1.00% |

**Table 5**

| **Dentifrice formulations** | | | | |
|---|---|---|---|---|
| Raw material | | | | |
| DI Water | 36.95% | 40.95% | 40.33% | 40.96% |
| Hydrous Sodium Lithium Magnesium Silicate | 0.50% | 0.50% | 0.75% | 0.50% |
| Sodium carboxymethylcellulose | 0.38% | 0.38% | 0.50% | 0.13% |
| Xanthan gum | | | | |
| Carrageenan Benvisco LB-2105 | | | | |
| Abrasive Silica | 10% | 10% | 10% | 10% |
| Thickening Silica | 8% | 4% | 4% | 4% |
| Sodium Fluoride | | | 0.25% | 0.25% |
| Glycerine | 10% | 10% | 10% | 10% |
| Sorbitol | 30% | 30% | 30% | 30% |
| Sodium Saccharin | 0.30% | 0.30% | 0.30% | 0.30% |
| Tetrasodiumpyrophosphate | 0.03% | 0.03% | 0.03% | 0.03% |
| Sodium Hydroxide | 0.06% | 0.06% | 0.06% | 0.06% |
| Sodium Lauryl Sulphate | 1.57% | 1.57% | 1.57% | 1.57% |
| Cocamidopropyl Betaine | 0.70% | 0.70% | 0.70% | 0.70% |
| D-Glucopyranoside, C10-16 Alkyl, Oligomeric | 0.51% | 0.51% | 0.51% | 0.51% |
| Phorphoric Acid | | | | |
| Monobasic Sodium Phosphate (USP)(Anhydrous) | | | | |
| Disodium Edetate USP | | | | |
| Hydrogen Peroxide 35% (as supplied) | | | | |
| Calcium Peroxide | | | | |
| Nipagin M | | | | |
| Niipasol M | | | | |
| Glycosperse L-20 | | | | |
| Flavor | 1.00% | 1.00% | 1.00% | 1.00% |

All of the above formulations described in the above examples can be used in a dispensing brush system. Each of the formulations has been shown acceptable to consumer, and each of the formulations has been shown to be stable with time through accelerated aging studies.

The optimal rheology of the oral care composition will depend in part on the type of dispensing toothbrush used. For example, the thickness and shear-thinning characteristics may depend on the robustness of the mechanical pump, with more robust pumps tolerating higher viscosity and/or less shear-thinning compositions. A number of embodiments of the invention have been described.

## Claims

1. An oral care composition comprising:
a) a carrier, and, dispersed in the carrier;
b) hydrous sodium lithium magnesium silicate;
c) sodium carboxymethylcellulose;
d) sodium lauryl sulphate;
e) cocamidopropyl betaine; and
f) D-glucopyranoside C₁₀₋₁₆ alkyl oligomeric.

2. The oral care composition of Claim 1 comprising:
a) 0.1 to 1.0% hydrous sodium lithium magnesium silicate;
b) 0.1 to 1.0% sodium carboxymethylcellulose;
c) 1.0 to 2.0% sodium lauryl sulphate;
d) 0.2 to 1.2% cocamidopropyl betaine; and
e) 0.1 to 1.0% D-glucopyranoside C₁₀₋₁₆ alkyl oligomeric.

3. The oral care composition of Claim 2 comprising:
a) 0.4 to 0.6 % hydrous sodium lithium magnesium silicate;
b) 0.35 to 0.40% sodium carboxymethylcellulose;
c) 1.5 to 1.6% sodium lauryl sulphate;
d) 0.6 to 0.8% cocamidopropyl betaine; and
e) 0.4 to 0.6% D-glucopyranoside C₁₀₋₁₆ alkyl oligomeric.

4. The oral care composition of Claim 1 further comprising a thickening silica.

5. The oral care composition of Claim 4 comprising between 4% and 8% of the thickening silica.

## Patentansprüche

1. Mundpflegezusammensetzung, umfassend:
a) einen Träger und, dispergiert in dem Träger,
b) wasserhaltiges Natriumlithiummagnesiumsilikat,
c) Natriumcarboxymethylcellulose,
d) Natriumlaurylsulfat,
e) Cocamidopropylbetain und
f) oligomeres D-Glucopyranosid-C₁₀₋₁₆-Alkyl.

2. Mundpflegezusammensetzung nach Anspruch 1, umfassend:
a) 0,1 bis 1,0 % wasserhaltiges Natriumlithiummagnesiumsilikat,
b) 0,1 bis 1,0 % Natriumcarboxymethylcellulose,
c) 1,0 bis 2,0 % Natriumlaurylsulfat,
d) 0,2 bis 1,2 % Cocamidopropylbetain und
e) 0,1 bis 1,0 % oligomeres D-Glucopyranosid-C₁₀₋₁₆-Alkyl.

3. Mundpflegezusammensetzung nach Anspruch 2, umfassend:
a) 0,4 bis 0,6 % wasserhaltiges Natriumlithiummagnesiumsilikat,
b) 0,35 bis 0,40 % Natriumcarboxymethylcellulose,
c) 1,5 bis 1,6 % Natriumlaurylsulfat,
d) 0,6 bis 0,8 % Cocamidopropylbetain und
e) 0,4 bis 0,6 % oligomeres D-Gglucopyranosid-C₁₀₋₁₆-Alkyl.

4. Mundpflegezusammensetzung nach Anspruch 1, ferner umfassend ein verdickendes Siliziumdioxid.

5. Mundpflegezusammensetzung nach Anspruch 4, das verdickende Siliziumdioxid zu zwischen 4 % und 8 % umfassend.

## Revendications

1. Composition de soins bucco-dentaires comprenant :
a) un véhicule et, dispersé dans le véhicule ;
b) du silicate de sodium, lithium et magnésium hydrique ;
c) de la carboxyméthylcellulose de sodium ;
d) du sodium-lauryl sulfate ;
e) de la cocamidopropyl bétaïne ; et
f) du D-glucopyranoside alkyle en C_{10 à 16} oligomère.

2. Composition de soins bucco-dentaires selon la revendication 1, comprenant :
a) 0,1 à 1,0 % de silicate de sodium, lithium et magnésium hydrique ;
b) 0,1 à 1,0 % de carboxyméthylcellulose de sodium ;
c) 1,0 à 2,0 % de sodium-lauryl sulfate;
d) 0,2 à 1,2 % de cocamidopropyl bétaïne ; et
e) 0,1 à 1,0 % de D-glucopyranoside alkyle en C_{10 à 16} oligomère.

3. Composition de soins bucco-dentaires selon la revendication 2, comprenant :
a) 0,4 à 0,6 % de silicate de sodium, lithium et magnésium hydrique ;
b) 0,35 à 0,40 % de carboxyméthylcellulose de sodium ;
c) 1,5 à 1,6 % de sodium-lauryl sulfate ;
d) 0,6 à 0,8 % de cocamidopropyl bétaïne ; et
e) 0,4 à 0,6 % de D-glucopyranoside alkyle en C_{10 à -16} oligomère.

4. Composition de soins bucco-dentaires selon la revendication 1, comprenant en outre une silice épaississante.

5. Composition de soins bucco-dentaires selon la revendication 4, comprenant entre 4 % et 8 % de la silice épaississante.
